# EUROPEAN PATENT SPECIFICATION

(11) **EP 3 544 982 B1**
(45) Date of publication and mention of the grant of the patent: **20.10.2021**
(21) Application number: 17812068.9
(22) Date of filing: 20.11.2017
(51) Int. Cl.: C07D 487/04

(54) **CHEMICAL PROCESS FOR PREPARING IMIDAZOPYRROLIDINONE DERIVATIVES AND INTERMEDIATES THEREOF**
CHEMISCHES VERFAHREN ZUR HERSTELLUNG VON IMIDAZOPYRROLIDINONDERIVATEN UND ZWISCHENPRODUKTEN DAVON
PROCÉDÉ CHIMIQUE DE PRÉPARATION DE DÉRIVÉS D'IMIDAZOPYRROLIDINONE ET DE LEURS INTERMÉDIAIRES

(30) Priority: 22.11.2016 WO PCT/CN2016/106767
(43) Date of publication of application: 02.10.2019
(73) Proprietor: Novartis AG, 4056 Basel (CH)
(72) Inventor: WANG, Hao, Changshu City Jiangsu 215537 (CN); YE, Jiong, Changshu City Jiangsu 215537 (CN); HAN, Bo, Changshu City Jiangsu 215537 (CN); FLUBACHER, Dietmar, 4002 Basel (CH); STETTLER, Hans, 4002 Basel (CH); GALLOU, Fabrice, 4002 Basel (CH); MALLET, Franck, 4002 Basel (CH); NAPP, Matthias, 4002 Basel (CH); HALLER, Michael, 4002 Basel (CH)
(74) Representative: Petersen, Holger
(86) International application number: PCT/IB2017/057263
(87) International publication number: WO 2018/096440

(56) References cited:
- WO-A1-2013/111105

## Description

### FIELD OF THE DISCLOSURE

The present disclosure is in the field of organic synthesis and relates to novel process steps and intermediates useful for the preparation of imidazopyrrolidinone derivatives such as (*6S*)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1*H*)-one, or a co-crystal thereof, or a solvate including hydrate thereof, and/or intermediates thereof.

### BACKGROUND OF THE DISCLOSURE

The present disclosure relates to a process for the preparation of imidazopyrrolidinone derivatives and their derivatives.

More particularly the present disclosure relates to a process for the preparation of the compound of formula (I) 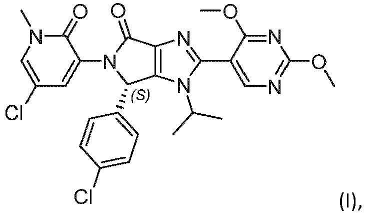 also referred to as (*6S*)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(*1H*)-one, or a co-crystal thereof, or a solvate including hydrate thereof, which are capable of inhibiting the interaction between tumor suppressor protein p53 or variants thereof, and MDM2 and/or MDM4 proteins, or variants thereof, respectively, especially binding to MDM2 and/or MDM4 proteins, or variants thereof.

Protein p53 is known as a tumor suppressor protein which helps to control cellular integrity and prevents the proliferation of permanently damaged cells by initiating, among other responses, growth arrest or apoptosis (controlled cell death). p53 mediates its effects in that it is a transcription factor capable of regulating a number of genes that regulate *e.g.* cell cycle and apoptosis. Thus, p53 is an important cell cycle inhibitor. These activities are tightly controlled by MDM2, an important negative regulator of the p53 tumor suppressor. "MDM2" (originally from the oncogene "murine double minute 2") refers both to the name of the gene as well as the protein encoded by that gene. The MDM2 protein functions acts both as an E3 ubiquitin ligase that recognizes the *N*-terminal transactivation domain (TAD) of the p53 tumor suppressor and thus mediates the ubiquitin-dependent degradation of p53, and as an inhibitor of p53 transcriptional activation.

The original mouse oncogene, which codes for the MDM2 protein, was originally cloned from a transformed mouse cell line. The human homologue of this protein was later identified and is sometimes also called HDM2 (for "human double minute 2"). Further supporting the role of MDM2 as an oncogene, several human tumor and proliferative disease types have been shown to have increased levels of MDM2, including *inter alia* soft tissue sarcomas, bone cancer (*e.g.* osteosarcomas), breast tumors, bladder cancer, Li-Fraumeni syndrome, brain tumor, rhabdomyosarcoma and adrenocortical carcinoma and the like. Another protein belonging to the MDM2 family is MDM4, also known as MDMX.

Dysregulation of the MDM2/p53 ratio, *e.g*. due to mutations, polymorphisms or molecular defects in the affected cells, can thus be found in many proliferative diseases. MDM2, in view of its mentioned effects, is capable to inhibit the activity of the tumor suppressor protein p53, thus leading to loss of p53's tumor suppressor activity and inhibiting regulatory mechanisms that impede cells from uncontrolled proliferation. As a consequence, uncontrolled proliferation can take place, leading to cancers such as tumors, leukemias or other proliferative diseases.

The imidazopyrrolidinone derivatives, such as compound of formula (I), or a co-crystal thereof, or a solvate including hydrate thereof, are described in WO2013/111105, in particular in examples 101 to 103. The compound of formula (I), so called (*6S*)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(*1H*)-one, is also known under the name *(S)*-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-*1H*-pyrrolo[3,4-d]imidazol-4-one. Those derivatives are capable of interfering with the interaction between p53 and MDM2 or especially oncogenic variants thereof and that thus allow p53 to exert its beneficial effect against uncontrolled tumor growth, allowing it *e.g.* to accumulate, to arrest the cell cycle and/or to cause apoptosis of affected cells. The imidazopyrrolidinone derivatives also show inhibition of the MDM2 / p53 and/or MDM4 / p53 interaction (this term including in particular Hdm2 / p53 and Hdm4 / p53 interaction), and in particular potent inhibition of the MDM2 / p53 interaction. In particular, the derivatives act as inhibitors of MDM2 interaction with p53 by binding to MDM2, and / or act as inhibitors of MDM4 interaction with p53 by binding to MDM4, rendering those derivatives useful in the treatment of a number of disorders, such as proliferative diseases, especially cancer.

Chemical processes are usually carried out first on a small scale during the research/early development phase. As development continues the scale is successively increased to finally reach the full size production scale in late phase development. Upon scaling up a process, topics related to process safety and efficacy are becoming more and more important. Failure to scale up properly may lead to the loss of process control, to accidents, such as unexpected exothermic reactions (runaway reactions), to health hazards when handling large amounts of hazardous and/or toxic chemicals, to environmental hazards, or to uneconomical use of chemicals. First processes for the preparation of the imidazopyrrolidinone derivatives as developed during the research/early phase are described in WO2013/111105. Nevertheless, there remains a need to provide improved processes for the preparation of the imidazopyrrolidinone derivatives, such as compound of formula (I), or a co-crystal thereof, or a solvate including an hydrate thereof, and/or intermediates thereof, which is economically efficient and safe and suitable for full size production scale.

The present disclosure is directed to an improved synthesis of compound of formula (I), or a co-crystal thereof, or a solvate including hydrate thereof, and its intermediates, using less hazardous chemicals and/or reaction conditions, generating less waste and providing a reproducible process that is easier to handle on a larger scale, a process that is more efficient and generates better quality compounds.

### BRIEF DESCRIPTION OF FIGURES

Figure 1 shows the X-ray powder diffraction pattern for compound of formula (D9).
Figure 2 shows the X-ray powder diffraction pattern for compound of formula (D10) L-malic acid.
Figures 3 to 7 show the proton-NMR spectra of the compounds D6, D7, D9, D10, and D14.

### DESCRIPTION OF THE DISCLOSURE

Increasing the amount of reactants and solvents in order to scale up the processes as described in WO2013/111105 to a full size commercial production may be associated with some risks such as loss of process control, unexpected exothermic reactions, accidents and safety issues while handling large amount of hazardous and/or toxic chemicals.

Surprisingly, it was found that modifying the process as described in WO2013/111105 to synthesize compound of formula (I), or a co-crystal thereof, or a solvate including hydrate thereof, and the synthetic intermediates in a way as disclosed herein provides a scalable method that can safely be handled on a larger scale with reproducible yields, less hazardous / toxic chemicals and produces less waste. In addition, this process produces more efficiently better quality compounds, at a lower cost. A summary of the process is showed in Scheme 1, *vide infra.*

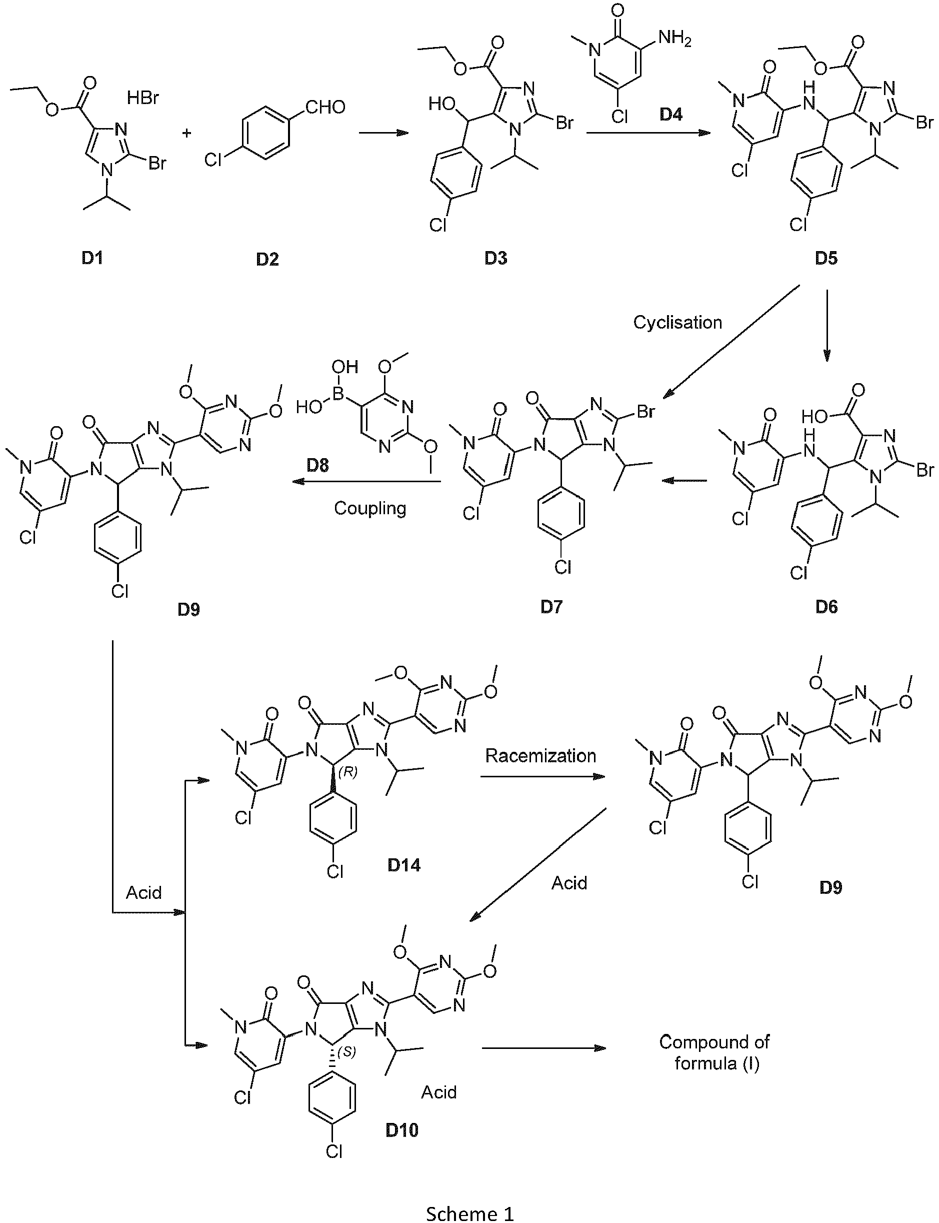

### Cyclisation Step: D5->(D6)->D7

The present invention relates to a process for preparing a compound of formula (D7), or a salt thereof, or a solvate thereof, 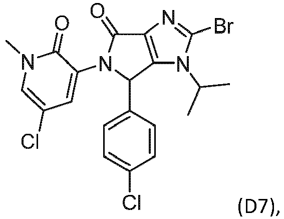 comprising the step of reacting a compound of formula (D6), or a salt thereof, or a solvate thereof, 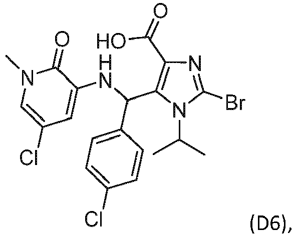 in a solvent in the presence of base and a coupling agent, wherein the coupling agent is N,N'-carbonyldiimidazole.

The process may be as defined in Scheme 2, comprising a two-step procedure including as first step the reaction of a compound of formula (D5), or a salt thereof, or a solvate thereof, as described in WO2013/111105, to obtain a compound of formula (D6). In a second step, the compound of formula (D6), or a salt thereof, or a solvate thereof, is reacted in a solvent in the presence of base and a coupling agent to give the compound of formula D7.

The reaction is preferably heated. Suitable solvents used for the reaction can be any polar solvents. For example, the solvent is one or more solvent(s) selected from tetrahydrofuran (THF), dimethylformamide (DMF), dimethylacetamide (DMAc), *N*-methyl-2-pyrrolidone (NMP), *N*-butyl-2-pyrrolidone, dichloromethane (DCM), acetonitrile, ethanol, methanol, ethyl acetate, *n*-propanol, 2-propanol, *n-*butanol, 2-butanol, *tert*-butanol, and 2-methyl-tetrahydrofuran. The preferred solvent in this process is one or more solvent(s) selected from tetrahydrofuran, dimethylformamide, dimethylacetamide, *N-*methyl-2-pyrrolidone, dichloromethane, acetonitrile, ethyl acetate and ethanol. Most preferably the solvent is tetrahydrofuran.

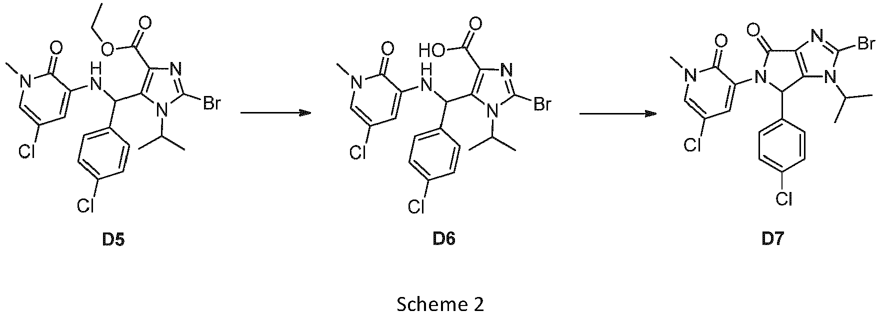

The base used to perform the reaction may be any base that a skilled person would select based on organic chemistry textbooks for this type of chemical transformation. The base can be for example potassium *tert*-butoxide (*t*BuOK), lithium bis(trimethylsilyl)amide (LiHMDS), sodium bis(trimethylsilyl)amide (NaHMDS), sodium methoxide (MeONa), potassium methoxide (KOMe), sodium ethoxide (EtONa), potassium ethoxide (KOEt), sodium *tert*-butoxide (*t*BuONa), n-butyllithium (*n*BuLi), Lithium diisopropylamine (LDA), sodium carbonate (Na₂CO₃), potassium carbonate (K₂CO₃), cesium carbonate (Cs₂CO₃), potassium phosphate tribasic (K₃PO₄), sodium phosphate tribasic (Na₃PO₄), *N,N-*di*iso*propylethylamine (DIPEA), triethylamine (Et₃N), sodium acetate (NaOAc), potassium acetate (KOAc), *N*-methylmorpholine (NMM) and 4-Dimethylaminopyridine (DMAP), or mixtures thereof. The preferred base in this process is one or more bases selected from *t*BuOK, LiHMDS, NaHMDS, MeONa, EtONa, *t*BuONa, *n*-BuLi, LDA, K₂CO₃, Cs₂CO₃, K₃PO₄, DIPEA, NMM, DMAP, KOMe, KOEt and Et₃N. Most preferably the reaction is performed in the presence of *t*BuOK. The base may be present in an amount between 0.01 to 0.5 equivalents. Preferably, the base may be present in a catalytic amount between 0.05 to 0.2 equivalents. Most preferably the base may be present in a catalytic amount of about 0.1 equivalent.

The cyclisation of compound (D6), or a salt thereof, as described in Scheme 2, is preferably done by stirring the reaction mixture for more than 2 hours, with tetrahydrofuran , potassium *tert*-butoxide and *N,N'-*carbonyldiimidazole. Preferably the reaction is performed at a temperatures between 50 to 80 °C, more preferably at a temperatures between 60 to 70 °C. Performing the reaction in tetrahydrofuran, in the presence of a catalytic amount of base (e.g. 0.05 to 0.2 equivalents), and in the presence of a coupling agent is preferred as the reaction is then more efficient, the yield is improved, the reaction leads to less impurities, and the risk of side reactions such as halogen exchange is reduced.

When salts are referred to herein, it is meant especially pharmaceutically acceptable salts or other generally acceptable salts, unless they would be excluded for chemical reasons, which the skilled person will readily understand. Salts can be formed with final products or intermediates where salt forming groups, such as basic or acidic groups, are present that can exist in dissociated form at least partially, *e.g*. in a pH range from 4 to 10 in aqueous solutions, or can be isolated especially in solid, especially crystalline, form. Such salts are formed, for example, as acid addition salts, preferably with organic or inorganic acids, from compounds or any of the intermediates mentioned herein with a basic nitrogen atom (*e.g*. imino or amino), especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, lactic acid, fumaric acid, succinic acid, citric acid, amino acids, such as glutamic acid or aspartic acid, maleic acid, hydroxymaleic acid, methylmaleic acid, benzoic acid, methane- or ethane-sulfonic acid, ethane-1,2-disulfonic acid, 1,5-naphthalene-disulfonic acid, 2-naphthalenesulfonic acid, benzenesulfonic acid, *N-*cyclohexylsulfamic acid, *N*-methyl-, *N*-ethyl- or *N*-propyl-sulfamic acid, or other organic protonic acids, such as ascorbic acid.

### Coupling Step: D7+D8->D9

The invention also optionally includes a step of preparing a compound of formula (D9), or a salt thereof, or a solvate thereof, in a process comprising the step of reacting a compound of formula (D7), or a salt thereof, or a solvate thereof as shown in Scheme 3, 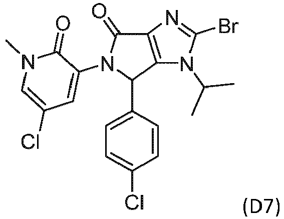 with a boronic acid of formula (D8), 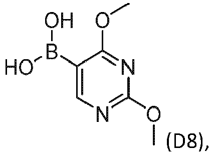 in a solvent in the presence of a metal-catalyst, a base and optionally a ligand, wherein the solution of (D8) is added to a solution of (D7) over a period of 2 to 8 hours,

Preferably the solution of (D8) is added over a period of 3 to 7 hours, preferably over a period of 4 to 6 hours. Most preferably the solution of (D8) is added over a period of 5 hours.

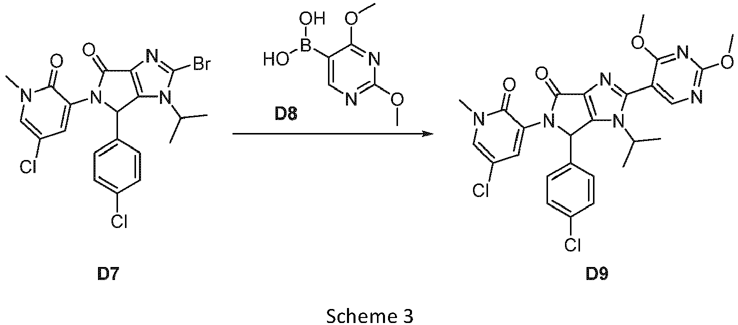

In general, the term "catalyst" refers to a catalytic amount of a chemical agent that enhances the rate of a chemical reaction by lowering the activation energy for the chemical reaction. The catalyst may be a heterogeneous catalyst or a homogenous catalyst. The catalyst may be generally present in an amount up to 10.0 mol%. Typically, the catalyst may be present in an amount below 6.0 mol%. The catalyst can be further present in a range from about 0.005 mol% to about 5.0 mol%, about 0.01 mol% to about 1.0 mol%, or from about 0.05 mol% to about 0.5 mol%, based on the starting material.

The term "heterogeneous catalyst" refers to a catalyst supported on a carrier, typically although not necessarily a substrate comprised of an inorganic material, for example, a porous material such as carbon, silicon and / or aluminum oxide. The term "homogeneous catalyst" refers to a catalyst that is not supported on a carrier.

The catalyst used to perform the reaction outlined in Scheme 3 may be any metal-catalyst that a skilled person would select based on organic chemistry textbooks for Suzuki coupling reactions. The metal-catalyst may be for example, selected from a group consisting of Pd(PPh₃)₂Cl₂, Pd(PPh₃)₄, Pd(dba)₂, Pd₂(dba)₃, PdCl₂, Palladium(II) acetate (Pd(OAc)₂), [Pd(allyl)Cl]₂, Pd(dppf)Cl₂, PdBr₂(PtBu₃)₂, Pd (crotyl)(PtBu₃)Cl, Pd(PtBu₃)₂, Pd(Amphos)₂Cl₂, Pd(allyl)(Amphos)Cl, Pd(Binap)Br₂, Pd(dcpp)Cl₂, Pd(DiPrPF)Cl₂, Pd-PEPPSI-IPr, Chloro(2-dicyclohexylphosphino-2',4',6'-tri*iso*propyl-1,1'-biphenyl)[2-(2-aminoethyl)phenyl)]palladium(II) (also known as XPhos Precatalyst 1st Generation), Chloro-(2-dicyclohexylphosphino-2',4',6'-tri*i*sopropyl-1,1'-biphenyl)[2-(2'-amino-1,1'-biphenyl)] palladium(II) (also known as XPhos Precatalyst 2nd Generation), Chloro(2-dicyclohexylphosphino-2',6'-dimethoxy-1,1'-biphenyl)[2-(2-aminoethylphenyl)]palladium(II) (also known as SPhos Precatalyst 1st Generation), Chloro(2-dicyclohexylphosphino-2',6'-dimethoxy-1,1'-biphenyl)[2-(2'-amino-1,1'-biphenyl)]palladium(II) (also known as XPhos Precatalyst 2nd Generation), Chloro(2-dicyclohexylphosphino-2',6'-diisopropoxy-1,1-biphenyl)[2-(2-aminoethylphenyl)]palladium(II) (also known as RuPhos Precatalyst 1st Generation), Chloro(2-dicyclohexylphosphino-2',6'-diisopropoxy-1,1'-biphenyl)[2-(2'-amino-1,1'-biphenyl)]palladium(II) (also known as RuPhos Precatalyst 2nd Generation), Pd/C, Pd, Ni(acaC)₂, NiCl₂, Ni(PPh₃)₂Cl₂, Ni(cod)₂, Ni(dppf)(cod), Ni(dppf)(cinnamyl), Ni(dppf)₂, Ni(dppf)Cl₂, Ni(dppp)Cl₂, Ni(PCy₃)₂Cl₂, Ni(dppe)Cl₂, [1,1'-Bis(di-*tert*-butylphosphino)ferrocene]dichloropalladium(II) (Pd(dtbpf)Cl₂), 1,1'-Bis(di-*is*opropylphosphino)ferrocene palladium dichloride (Pd(dippf)Cl₂), or mixtures thereof. The preferred metal-catalyst in this process is one or more a palladium-catalyst selected from [1,1'-Bis(di-*tert-*butylphosphino)ferrocene]dichloropalladium(II) (Pd(dtbpf)Cl₂), 1,1'-Bis(di-*is*opropylphosphino)ferrocene palladium dichloride (Pd(dippf)Cl₂), [1,1'-Bis(diphenylphosphino)ferrocene]dichloropalladium(II) Pd(dppf)Cl₂ and Pd(OAc)₂. Most preferably the metal-catalyst agent is [1,1'-Bis(diphenylphosphino)ferrocene]dichloropalladium(II) (Pd(dppf)Cl₂). The catalyst may be present in an amount between 0.1 to 6 mol%. Preferably, the metal-catalyst is present in an amount between 4 to 6 mol%. Most preferably the amount is about 5 mol%.

The reaction as described in Scheme 3 may also be performed in a presence of a ligand. Preferably, the reaction is performed in the presence of a ligand. The term "ligand" means any compound, achiral or chiral, that can form a complex with a transition metal. The ligand used to perform the reaction is any ligand that a skilled person would select based on organic chemistry textbooks for Suzuki coupling reactions. The ligand may be one or more ligands selected from the group of PPh₃, P(*o*Tol)₃, P(*o*Tol)Ph₂, P(*p*Tol)₃, P*t*Bu₃, P*t*Bu₃*HBF₄, PCy₃, PCy₃*HBF₄, P(O*i*Pr)₃, DPE-Phos, dppf, dppe, dppp, dcpp, dppb, P(Furyl)₃, CPhos, SPhos, RuPhos, XPhos, DavePhos, JohnPhos and Xantphos. The ligand may be present in a range from about 2 mol% to about 10 mol%. Preferably the ligand is present in an amount of about 5 mol%.

The base used to perform the reaction as described in Scheme 3 is any base that a skilled person would select based on organic chemistry textbooks for Suzuki coupling reactions. The base may be for example sodium carbonate (Na₂CO₃), potassium carbonate (K₂CO₃), cesium carbonate (Cs₂CO₃), thallium(I) carbonate (Tl₂CO₃), sodium bicarbonate (NaHCO₃), potassium bicarbonate (KHCO₃), sodium acetate (NaOAc), potassium acetate (KOAc), sodium phosphate (Na₃PO₄), potassium phosphate (K₃PO₄), lithium hydroxide (LiOH), sodium hydroxide (NaOH), potassium hydroxide (KOH), cesium hydroxide (CsOH), barium hydroxide (Ba(OH)₂), sodium methoxide (NaOMe), potassium methoxide (KOMe), sodium ethoxide (NaOEt), potassium ethoxide (KOEt), thallium ethoxide (TIOEt), sodium phenoxide (NaOPh), trimethylamine (Et₃N), *N,N*-diisopropylethylamine (DIPEA), sodium tert-butoxide (NaOtBu), potassium tert-butoxide (KOtBu), potassium fluoride (KF) and cesium fluoride (CsF), or mixtures thereof. The preferred base in this process is one or more bases selected from KF, K₃PO₄, NaOH, and KHCO₃. Most preferably, the reaction is performed in the presence of KF.

The reaction described in Scheme 3 may be performed in a solvent selected for example from tetrahydrofuran, 1,4-dioxane, 2-methyltetrahydrofuran, acetonitrile, toluene, dimethylformamide, dimethylacetamide, dimethylsulfoxide, dimethoxyethane, dichloromethane, acetone, *N*-methyl-2-pyrrolidone, *N*-butyl-2-pyrrolidone, dimethylcarbonate, ethylacetate, *is*opropylacetate, *tert*butylacetate, pentane, hexane, heptane, anisole, pyridine, triethylamine, water, methanol, ethanol, *n*-propanol, 2-propanol, *n*-butanol, 2-butanol, *is*opropanol, *tert*-butanol and butanone, or mixtures thereof. The preferred solvent in this process is one or more solvents selected from 1,4-dioxane, water, dimethoxyethane, *n*-butyl-2-pyrrolidone and butanone. Most preferably the solvent is a mixture of 1,4-dioxane and water. The ratio (volume to volume) of said mixture may be in the range from 20:1 to 1:20, preferably the ratio is such that there is an excess of 1,4 dioxane over water, e.g. the ratio is in the range from 20:1 to 5:1, preferably the ratio is from 15:1 to 10:1.

The reaction as described in Scheme 3 is advantageously performed when the solvent is a mixture of 1,4-dioxane and water (e.g. in a ratio from 15:1 to 10:1), the base is potassium fluoride (KF), the palladium-catalyst is [1,1'-Bis(diphenylphosphino)ferrocene]dichloropalladium(II) (Pd(dppf)Cl₂), the boronic acid is (D8). Preferably, the reaction is performed at a temperature between 80 to 180°C, more preferably between 100 to 120 °C. Most preferably, the reaction is performed at 110 °C. Performing the reaction under those conditions is a particularly advantageous as the reaction is highly efficient, the yield is improved and the generation of by-products is reduced.

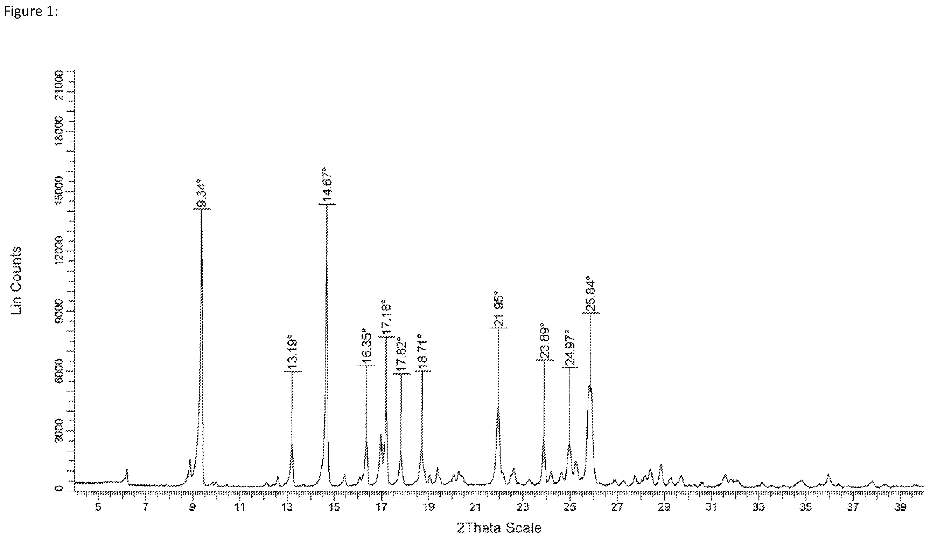

The X-ray powder diffraction (XRPD) of the substantially pure compound (D9) exhibits one or more, preferably at least 2 to 5, more preferably all, diffraction peaks having maxima at diffraction angles selected from 9.34°, 13.19°, 14.67°, 16.35°, 17.18°, 17.82°, 18.71°, 21.95°, 23.89°, 24.97°, 25.84° (2θ degrees, copper K alpha 1), as shown in Figure 1. More preferably, the XRPD of compound (D9) exhibits one or more, preferably 2 to 4, more preferably all, diffraction peaks having maxima at diffraction angles selected from 9.34°, 14.67°, 17.18°, 21.95°, 23.89°, 25.84° (2θ degrees, copper K alpha 1). The XRPD maxima values (in degrees) generally means ± 0.3°, more preferably ± 0.2°, and most preferably ± 0.1° of the given value.

The term "substantially pure" means that more than 80% of one crystalline form of a compound, or salt thereof, or co-crystal, as described herein, is present or isolated, preferably at least 85%, more preferably at least 90%, and most preferably at least 95% of one of the crystalline forms described herein is present or isolated.

### Stereoisomers separation and preparation of compound (D10)

The invention also optionally also includes a step of preparing a compound of formula (D10) as described in the reaction Scheme 4, comprising the step of reacting a compound of formula (D9), or a salt thereof, or a solvate thereof, in a solvent with an acid to obtain a mixture of compound of formula (D14), or a salt, co-crystal, or a solvate thereof, and compound of formula (D10) as described herein. Compound of formula (D10) as described herein and in Scheme 4 is a mixture of two compounds: an imidazopyrrolidinone derivative and an acid. Compound of formula (D10) can be in the form of a salt, a complex, a hydrate, a solvate, a polymorph, a co-crystal, or a mixture thereof. Preferably compound of formula (D10) is in the form of a co-crystal.

The invention also optionally includes a step of preparing a compound of formula (D10), as described herein, comprising the steps of dissolving a compound of formula (D9), or a salt thereof, or a solvate thereof, in a solvent with an acid to obtain a mixture of compound of formula (D14), or a salt thereof, or a solvate thereof and compound of formula (D10) as described herein. During the separation one of the stereoisomers precipitates from the solution in a compound form (D10) as described herein while the second stereoisomer remains in solution.

The process, as described in Scheme 4, is preferably performed with one or more acid(s) selected from L-malic acid, lactic acid, tartaric and malonic acid. More preferably the acid is L-malic acid. L-malic acid is also referred to as L-(-)-malic acid, (S)-(-)-2-hydroxysuccinic acid or L-hydroxybutanedioic acid. The separation described in Scheme 4 is preferably performed in one or more solvent(s) selected from, for example, ethyl acetate isopropyl acetatebutyl acetate, and propyl acetate. More preferably the solvent is ethyl acetate.

The separation of stereoisomers (D14) and (D10) as described in the scheme below is highly efficient, improves the yield of compound of formula (D10) as described herein and improves the overall cost of the synthesis.

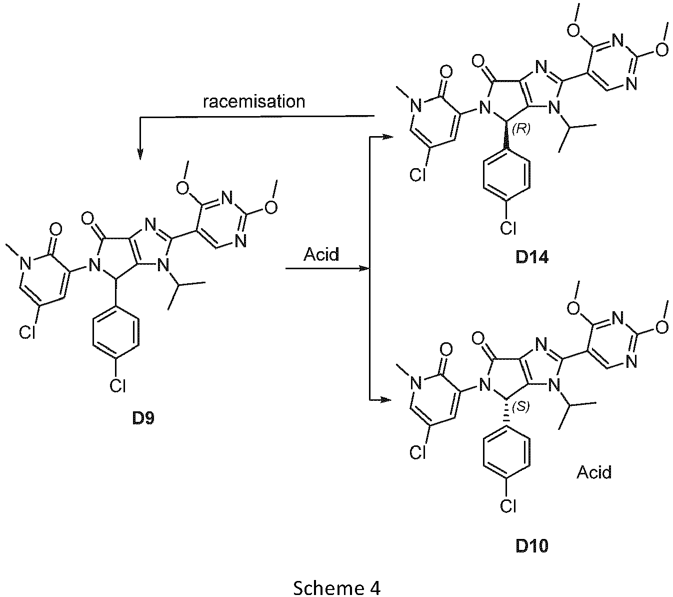

The term "stereoisomers" means one of the absolute configurations of a single organic molecule having at least one asymmetric carbon. Also, as used herein, the term refers to any of the various stereo isomeric configurations which may exist for a given compound of the present disclosure and includes geometric isomers. It is understood that a substituent may be attached at a chiral center of a carbon atom. Therefore, the disclosure includes enantiomers, diastereomers or racemates of the compound. "Enantiomers" are a pair of stereoisomers that are non-superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers or axes and may thus give rise to enantiomers, diastereomers, and other stereoisomeric forms that may be defined, in terms of absolute stereochemistry, as (R)- or (S)-. The present disclosure is meant to include all such possible isomers, including racemic mixtures, optically pure forms and intermediate mixtures.

To further increase the yield of compound of formula (D10), as described herein, the process for preparing the compound (D10) can optionally comprise the further steps of reacting (D14), or a salt, a co-crystal, or a solvate thereof, to obtain a compound of formula (D9), or a salt thereof, or a solvate thereof. Compound of formula (D9) may be obtained by dissolving compound of formula (D14) in one or more polar protic solvent(s) selected from, for example, formic acid, 1-pentanol, 2-pentanol, 3-pentanol, 1-hexanol, n-butanol, *is*opropanol, ethanol, methanol, acetic acid, and water. Preferably the solvent is methanol. The reaction may be performed in the presence of a base selected from, for example, sodium bicarbonate (NaHCO₃), sodium hydroxide (NaOH), potassium bicarbonate (KHCO₃) and potassium hydroxide (KOH). Preferably the base is sodium bicarbonate (NaHCO₃). Preferably the reaction is performs at a temperature between 50 to 80 °C, more preferably between 55 to 75 °C. Most preferably the reaction is performed at about 65 °C. Performing the reaction under those conditions is advantageous as it is highly efficient, improves the yield and reduces the generation of by-products.

Then the newly formed compound of formula (D9) or a salt thereof, or a solvate thereof, is reacted in a solvent in the presence of an acid to obtain a mixture of compound (D10), as described herein, and compound (D14) in the same manner as above. Optionally, the transformation of compound (D14) to compound (D10) via compound (D9), as described in Scheme 4, can be repeated one or more times, preferably the transformation is repeated at least one time.

Yet another aspect of the present disclosure relates to compound (D10), wherein the acid is selected from L-malic acid, lactic acid, tartaric acid and malonic acid. Preferably the acid is L-malic acid. Another aspect of the present disclosure relates to compound (D10) obtained by or obtainable by the process as shown in Scheme 4, wherein the acid is selected from L-malic acid, lactic acid, tartaric acid and malonic acid. Preferably the acid is L-malic acid.

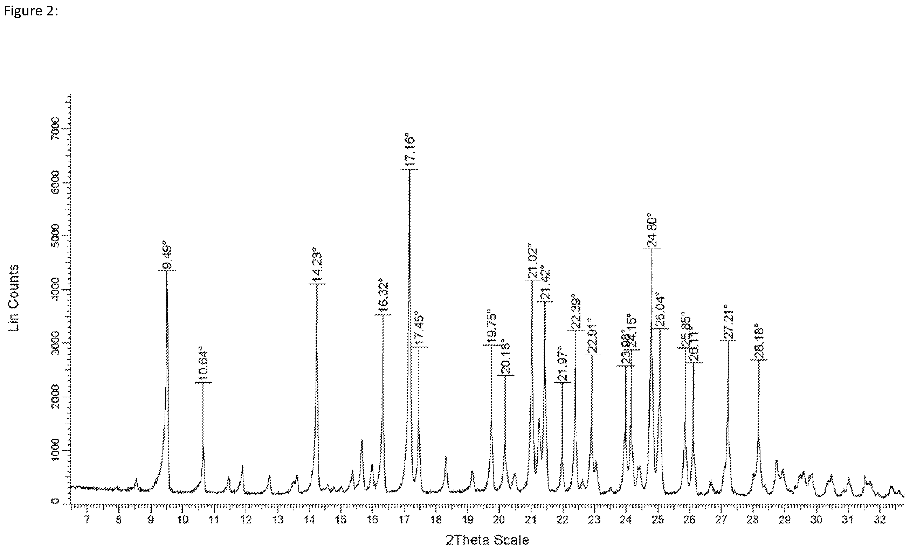

Preferably, compound of D10 is obtained as crystalline material. The X-ray powder diffraction (XRPD) of the substantially pure crystalline material of compound of formula (D10) L-malic acid exhibits one or more, preferably at least two to four, more preferably all, diffraction peaks having maxima at diffraction angles selected from 9.49°, 10.64°, 14.23°, 16.23°, 17.16°, 17.45°,19.75°, 20.18°, 21.02°, 21.42°, 21.97°, 22.39°, 22.91°, 23.98°, 24.15°, 24.80°, 25.04°, 25.85°, 26.11°, 27.21°, 28.18° (2θ degrees, copper K alpha 1). also as shown in Figure 2. Most preferably the XRPD of compound of formula (D10) L-malic acid exhibits one or more, preferable at least two to four, more preferably all diffraction peaks having maxima at diffraction angles selected from 9.49°, 14.23°, 16.23°, 17.16°, 21.02°, 21.42°, 22.39°, 24.80°, 25.04°, 27.21° (2θ degrees, copper K alpha 1). The XRPD maxima values (in degrees) generally means within ±0.3°, more preferably within ±0.2°, and most preferably within ±0.1° of the given value.

The compound (D10) L-malic acid also exhibits a melting point (mp) of 188 ±1°C (onset, by DSC).

The term "co-crystal" means a crystalline entity in which more than one molecular substance is incorporated into the unit cell, and both or all molecular components are solid at room temperature and pressure, each containing distinctive physical characteristics, such as structure, melting point and heats of fusion. Co-crystals are usually solids that are crystalline materials composed of two or more molecules in the same crystal lattice. Co-crystals can be composed of an active pharmaceutical Ingredient (API) with a neutral guest compound (also referred to as a conformer) in a crystal lattice. When the API and the co-crystal former are in a neutral state and they can be constructed or bonded together through non-ionic interactions, hydrogen bonds (non-covalent bond is formed between a hydrogen bond donor of one of the moieties and a hydrogen bond acceptor of the other), π-stacking, guest-host complexation and Van der Waals interactions. Various properties of a pharmaceutical composition may affect the onset of solid-state nucleation or precipitation of the API. Such properties include the identity or amount of the excipient and the identity or amount of the pharmaceutical compound in the composition. Other properties may include the amount of other diluents or carriers such as salts or buffering compounds. The pharmaceutical compound itself may be screened in a variety of different forms if it is capable of polymorphism. Additionally, different salt, solvate, hydrate, co-crystal and other forms of the API may be screened in accordance with the disclosure. Generally speaking, the API is typically capable of existing as a supersaturated solution, preferably in an aqueous-based medium. The API may be a free acid, or a free base, or a co-crystal, or salt, or a solvate, or a hydrate or a dehydrate thereof.

### Formation of compound of formula (I), or a solvate including hydrate thereof, or a co-crystal thereof.

The invention also optionally includes a step of preparing a compound of formula (I), or a solvate including a hydrate thereof, or a co-crystal thereof, as described in Scheme 5.

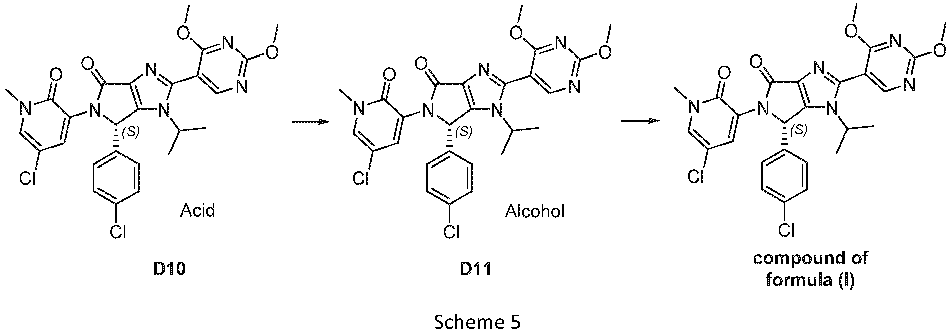

The reaction to form intermediate (D11), or a salt thereof, or a solvate thereof, or a co-crystal thereof as described in Scheme 5 may be performed in a solvent selected, for example, from dichloromethane, ethanol, ethyl acetate, isopropyl acetate and water, in the presence of an alcohol. Preferably the reaction is performed in ethanol, ethyl acetate and water, in the presence of ethanol. The reaction to form intermediate (D11), or a salt thereof, or a solvate thereof, or a co-crystal thereof is preferably performed at a temperature between 30 to 70°C, more preferably at a temperature of 40 to 60 °C, most preferably at a temperature of 50 °C.

Then intermediate (D11), or a salt thereof, or a solvate thereof, or a co-crystal thereof, is transformed into a compound of formula (I), or a solvate thereof, or a co-crystal thereof, as described in Scheme 5. The reaction is preferably performed in a solvent selected from, for example, water, acetone, methanol, ethanol, *iso*propanol and n-butanol, or a mixture thereof. More preferably the reaction is performed in water and acetone, or a mixture thereof. Compound of formula (I) is preferentially obtained in a substantially pure crystalline form.

The invention also optionally includes the step of forming a substantially pure crystalline form of compound of formula (I). The substantially pure crystalline form of compound of formula (I) can be obtained by spontaneous crystallization of the compound of formula (I).The present disclosure also relates to the formation of the substantially pure crystalline form of compound of formula (I) by adding a seed, i.e. a small amount of the crystalline form of compound of formula (I) obtained by spontaneous crystallization, as described above, (1% by weight or less, referred to as seeding) to, for example, a suspension, a solution, a mixture, or a dispersion, to enhance the crystallization. The temperature usefully employed for seeding ranges from 20 to 40 °C, more preferably at a temperature of 25 °C. As used herein, the term "seed" can be used as a noun to describe one or more crystals of a crystalline compound of formula (I). The term "seed" can also be used as a verb to describe the act of introducing said one or more crystals of a crystalline compound of formula (I) into an environment (including, but not limited to, for example, a solution, a mixture, a suspension, or a dispersion) thereby resulting in the formation of more crystals of the crystalline compound of formula (I).

The invention includes a process for preparing a compound of formula (I), or a solvate including hydrate thereof, or a co-crystal thereof, the process comprising the steps of: preparing a compound of formula (D7), or a salt thereof, or a solvate thereof, according to claim 1 and as described in Scheme 2 and reacting the obtained compound of formula (D7), or a salt thereof, or a solvate thereof, with a compound (D8) as described in Scheme 3 to obtain a compound of formula (D9), or a salt thereof, or a co-crystal thereof. Then preparing a compound of formula (D10) as defined herein and described in scheme 4 and finally reacting compound of formula (D10), defined herein, as described in Scheme 5 to obtain a compound of formula (I), or a solvate including an hydrate thereof, or a co-crystal thereof. Preferably the compound of formula (I) is prepared in its hydrate form, which is herein referred to as (D12).

### ABBREVIATIONS

- Ac: Acetyl
- acac: Acetylacetone
- Amphos: Bis(di-*tert*-butyl(4-dimethylaminophenyl)phosphine)
- AOP: 7-Azabenzotriazol-1-yloxytris(dimethylamino)phosphonium hexafluorophosphate
- API: Active pharmaceutical ingredient
- BEP: 2-Bromo-1-ethyl-pyridinium tetrafluoroborate
- BINAP: (1,1'-Binaphthalene-2,2'-diyl)bis(diphenylphosphine)
- BOP: Tri(dimethylamino)benzotriazol-1-yloxyphosphonium hexafluorophosphate
- BTFFH: Bis(tetramethylene)fluoroformamidinium hexafluorophosphate
- CDI: *N,N*'-Carbonyldiimidazole
- CMDT: 2-Chloro-4,6-dimethoxy-1,3,5-triazine
- cod: Cyclo-1,5-octadiene
- cy: Cyclohexyl
- dba: 1,4-Bis(diphenylphosphino)butane
- DCC: *N,N*'-Dicyclohexylcarbodiimide
- DCM: Dichloromethane
- dcpp: 1,3-Bis(dicyclohexylphosphanyl)propane
- DIPA: Diisopropylamine
- DIPEA: *N,N*-diisopropylethylamine
- DMAc: Dimethylacetamide
- DMAP: 4-Dimethylaminopyridine
- DMF: *N,N*-dimethylformamide
- DMTMM: 4-(4,6-Dimethoxy-1,3,5-triazin-2-yl)-4-methylmorpholinium chloride
- Dppb: 1,4-Bis(diphenylphosphino)butane
- dppe: 1,2-Bis(diphenylphosphino)ethane
- dppf: 1,1'-bis(diphenylphosphanyl)ferrocene
- dppp: 1,3-Bis(diphenylphosphanyl)propane
- EDC: *N*-Ethyl-*N*'-(3-dimethylaminopropyl)carboiimide
- Eq.: Equivalent
- Et₃N: Triethylamine
- EtONa/EtOK: Sodium ethoxide / Potassium ethoxide
- g: Gram(s)
- Ghosez's reagent: 1-Chloro-*N,N*,2-trimethylpropenylamine
- h: Hour(s)
- HATU: *N,N,N',N*'-Tetramethyl-O-(7-azabenzotriazol-1-yl)uronium hexafluorophosphate
- HBTU: *N,N,N',N*'-Tetramethyl-*O*-(*1H*-benzotriazol-1-yl)uronium hexafluorophosphate
- HDM2: Human double minute 2
- HOBt: hydroxybenzotriazole
- HPLC: High performance liquid chromatography
- IBCF: Isobutyl carbonochloridate
- K₂CO₃ / Na₂CO₃: Potassium carbonate / Sodium carbonate
- K₃PO₄/Na₃PO₄: Potassium phosphate tribasic / Sodium phosphate tribasic
- KF: Potassium fluoride
- KHCO₃ / NaHCO₃: Potassium hydrogen carbonate / Sodium hydrogen carbonate
- LDA: Lithium diisopropylamine
- LiHMDS: Lithium bis(trimethylsilyl)amide
- MDM2: Murine double minute 2
- MeONa: Sodium methoxide
- mg / mL: Milligram(s) / Milliliter(s)
- mol: Mole(s)
- Ms₂O: Methanesulfonic anhydride
- NaHMDS: Sodium bis(trimethylsilyl)amide
- NaOH: Sodium hydroxide
- *n*-BuLi: *n*-Butyllithium
- NMM: *N*-Methylmorpholine
- NMR: Nuclear magnetic resonance spectroscopy
- Pd(OAc)₂: Palladium(II) acetate
- Pd/C: Palladium on carbon
- Pd₂(dba)₃: Tris(dibenzyllideneacetone)dipalladium(0)
- Pd(dippf)Cl₂: 1,1' -Bis(diisopropylphosphino)ferrocene palladium dichloride
- Pd(dppf)Cl₂: 1,1' -Bis(diphenylphosphino)ferrocene palladium dichloride
- Pd(dtbpf)Cl₂: [1,1' -Bis(di-*tert*-butylphosphino)ferrocene]dichloropalladium(II)
- Pd(dtbpf)Cl₂: [1,1' -Bis(di-*tert*-butylphosphino)ferrocene]dichloropalladium(II) [1,3-Bis(2,6-di*iso*propylphenyl)imidazole-2-ydilene](3-chloropyridyl)palladium(II)
- Pd-PEPPSI-iPr: dichloride
- PEPPSI: Pyridine enhanced precatalyst preparation stabilization and initiation
- Phos: Phosphine
- POBr₃: Phosphoryl bromide
- POCl₃: Phosphoryl chloride
- PyBOP: Benzotriazol-1-yl-oxytripyrrolidinophosphonium hexafluorophosphate
- T°C: Temperature in degree Celsius
- T3P: 2,4,6-Tripropyl-1,3,5,2,4,6-Trioxatriphosphorinane-2,4,6-Trioxide
- TAD: Transactivation domain
- tBuOK: Potassium tert-butoxide
- tBuONa: Sodium *tert*-butoxide
- TFFH: Fluoro-*N,N,N',N*'-tetramethylformamidinium hexafluorophosphate
- THF: Tetrahydrofurane

### EXAMPLES

The Following examples are merely illustrative of the present disclosure and they should not be considered as limiting the scope of the invention which is defined by the accompanying claims.

### Synthesis

Generally, compounds according to the present disclosure can be synthesized by the route described in the Schemes 1 - 5 as shown herein.

The skilled person will appreciate that the general synthetic routes detailed above show common reactions to transform the starting materials as required. When specific reactions are not provided the skilled person will know that such reactions are well known to those skilled in the art and appropriate conditions considered to be within the skilled person's common general knowledge. The starting materials are either commercially available compounds or are known compounds and can be prepared from procedures described in the organic chemistry art.

Compounds as described herein, in free form, may be converted into salt form and vice versa, in a conventional manner understood by those skilled in the art. The compounds in free or salt form can be obtained in the form of hydrates or solvates containing a solvent used for crystallization. Compounds described herein can be recovered from reaction mixtures and purified in a conventional manner. Isomers, such as stereoisomers, may be obtained in a conventional manner, e.g. by fractional crystallization or asymmetric synthesis from correspondingly asymmetrically substituted, e.g. optically active, starting materials. The various starting materials, intermediates, and compounds of the preferred embodiments may be isolated and purified, where appropriate, using conventional techniques such as precipitation, filtration, crystallization, evaporation, distillation, and chromatography. Unless otherwise stated. Salts may be prepared from compounds by known salt-forming procedures.

The compounds described herein can be prepared, e.g. using the reactions and techniques described below and in the examples. The reactions may be performed in a solvent appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the disclosure.

It would be understood by the skilled person in the art, that the reactions were run on a small scale first in order to access if the starting materials could react in high yields and high purities before to be scalable. The desired compounds obtained during such small scale reaction, that spontaneously crystallized, were used to enhance the latest reactions, using the technique of "seeding". Here below approximately 1% by weight or less of seeding crystals were added, if needed, to the reaction mixture to generate quicker the spontaneous crystallization of the desired product.

### Measurements methods

- **Proton-NMR** analyses were performed using a Bruker 400 NMR machine.
- **XRPD** was measured using a Panalytical x-pert Pro and/or a Bruker D8 advanced equipment. Sample amount used for the measurement was between 20-200 mg. The sample preparation technique used was kapton foil spread with stirrer oil and the sample was held using a transmission sample holder: one way holder system for inner parts and metal ring for outer part. The measurement conditions were as follow: oscillation of 0.3sek/step, measuring time: time per step 0.017, tension kV40/mV40. Wave length used was copper K alpha1 radiation. The compound used for calibration was corundum [α-Al₂O₃]. The error on the measurement is ±0.2 theta.

It should be noted that different samples of a particular crystalline form will share the same major X-ray powder diffraction (XRPD) peaks, but that there can be variation in powder patterns with regard to minor peaks.
- **Melting point** was measured using the Mettler Toledo DSC821e equipment with Ceramic FRS5 Sensor. The purge gas was N₂; 40µl gold plated crucibles (high pressure). Conditions of the measurement: heating rate 4.0 K/min.

### Example 1: Cyclisation Step (D5->D6->D7)

### Process step D5->D6

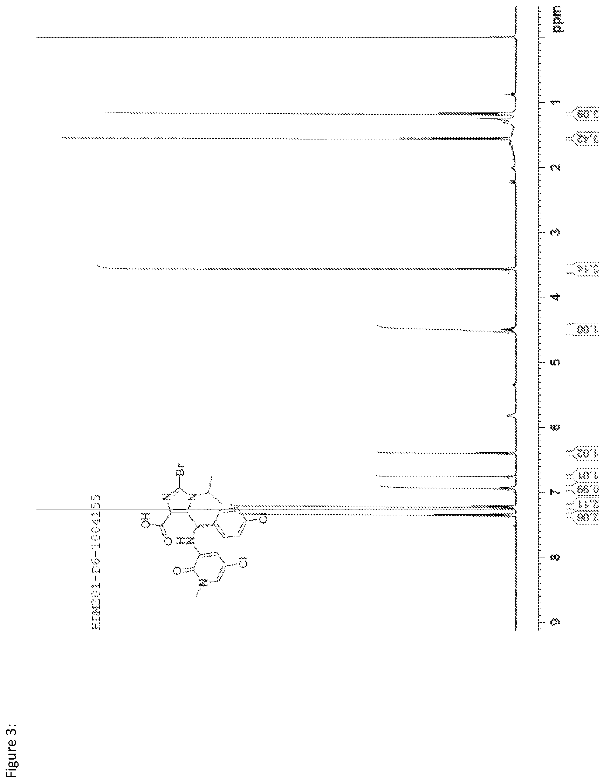

A suspension of compound D5 (60g), NaOH solid (5.3g, 1.2 eq) in water 180g and ethanol 370g was heated up to reflux and stirred for more than 2 hour. After conversion to D6, 3.1% Wt HBr aqueous (385g, 1.35 eq) was added slowly into the reaction mixture over 1 hour at reflux. The resulting suspension was stirred at reflux for additional 0.5 hour, cooled to 25 °C over 6 hours and stirred at 25 °C for 8 hours. The suspension was filtrated, washed with a 50% solution of ethanol, dried under vacuum at 60 °C to give compound D6 as a white solid (54.1g, yield 95%, 1H-NMR, 400 MHz, CDCl3: see Figure 3).

### Process step D6->D7

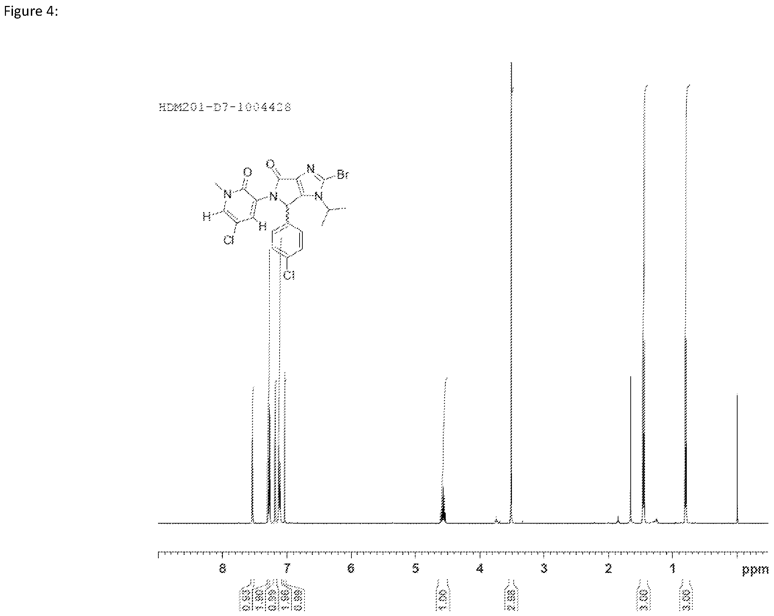

A suspension of containing compound D6 (70g) in 840g tetrahydrofuran (THF) was heated up to 35 - 45 °C. Then *N,N'*-Carbonyldiimidazole (CDI, 1.2 eq., 26.5g) was added portionwise. The mixture was then heated up to 60 - 70 °C and stirred for more than 1 hour. After conversion to the active intermediate, the reaction mixture was cooled to 55 - 60 °C, followed by slow addition of a 6% solution of potassium *tert-*butoxide in THF (0.1 eq.). The mixture was heated up again to 60 - 70 °C and stirred for more than 2 hours until the sum of compound D6 and the intermediate was less than 3%. The reaction mixture was then cooled down to 25 °C, quenched by addition of water (0.5 eq., 1.2g). The solvent was exchanged with ethanol by distillation under vacuum at a pressure of 250 - 300 mbar and at 70 °C. Then water was added into the distillation residue at 60 - 70 °C. After addition, the mixture was stirred further at 60 - 70 °C for 1 hour, cooled down to 25 °C over a period of 3 hours, and finally stirred again at 25 °C for another 2 hours. The reaction mixture was filtered, washed with a 50% solution of ethanol, dried under vacuum at 85 °C to give compound D7 as a white solid (65.7g, yield 97%, 1H-NMR, 400 MHz, CDCl3: see Figure 4).

### Example 2: Coupling Step (D7->D9)

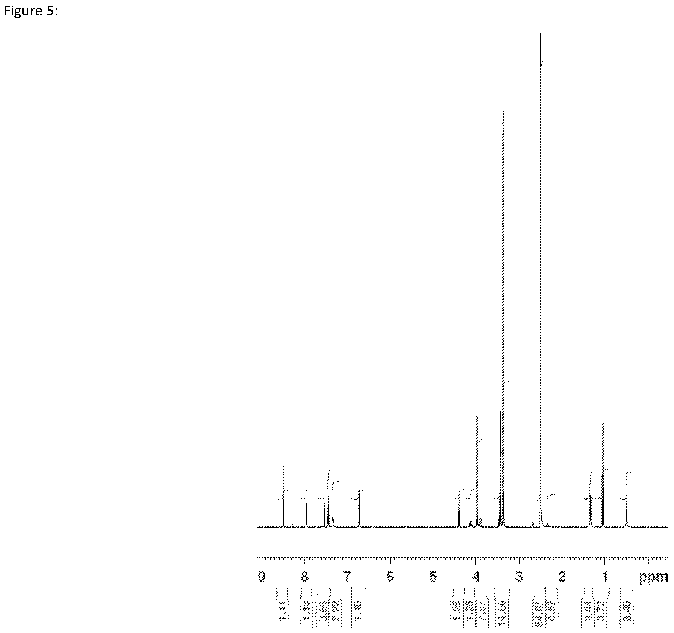

A suspension of boronic acid D8 (10.40 g, 1.375 eq) and potassium fluoride (KF, 9.37 g, 4 eq.) in 77 mL of water was warmed up to 35 °C in order to obtain a clear solution. A reactor was charged under Argon with Bromide D7 (20.00 g, 1.00 eq.), 12 mL water and 140 mL 1,4-dioxane. The mixture was refluxed and Pd(dppf)Cl₂ (1.475g, 0.05 eq.) was added. The aqueous solution of D8/KF was added within 75 min and the reaction was monitored by HPLC. The 1,4-dioxane was removed by distillation at 300 - 400 mbar. During the distillation 150 mL water was added, then 250 mL ethyl acetate was added and a biphasic mixture was obtained. The phases were separated and the organic layer was extracted with 100 mL half concentrated brine. Ethyl acetate was removed by distillation. To the resulting brown suspension 200 mL ethanol was added. Solvent was removed by distillation while keeping the volume constant by addition of 100 mL ethanol. After completion of the solvent switch, the mixture was cooled to 0 °C within 60 min. Seeding crystals obtained from a previous reaction were added. The mixture was stirred for 60 min at 0 °C and the product was collected by filtration. The product was dried under vacuum at 55 °C to obtain compound of formula D9 (Yield about 75 %, 1H-NMR, 400 MHz, d6-DMSO: see Figure 5).

### Example 3: Stereoisomers separation - (D9->D10+D14) and (D14->D9->D10+D14)

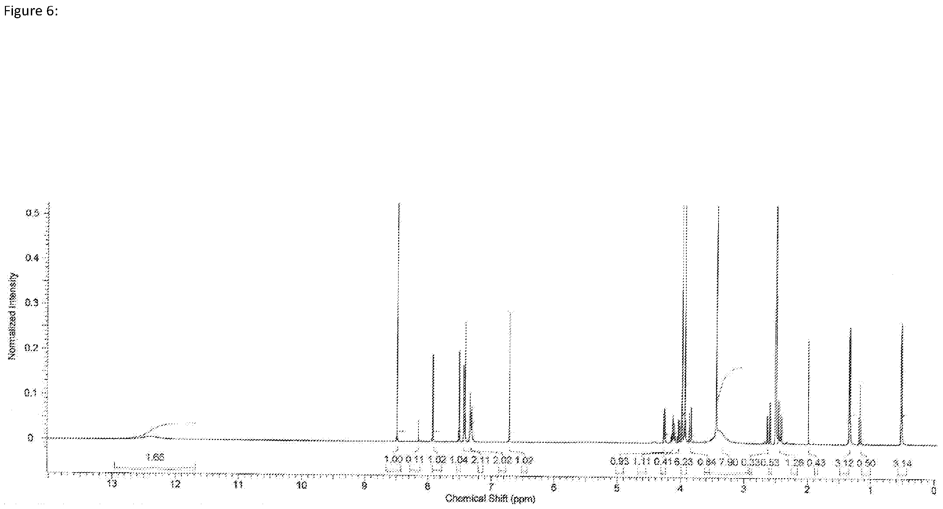

A solution of L-malic acid (7.10 g, 1 eq.) in 100 mL ethyl acetate was prepared at 70 °C and stored at 50 °C until further use. A reactor was charged with compound D9 (30.00 g, 1 .00 eq.) and 1000 mL ethyl acetate. 10.00 g water was added and the mixture was stirred at reflux. The remaining residue was removed by filtration. The reactor was charged with the solution of compound D9 in ethyl acetate and the solution of L-malic acid. Then water was removed by azeotropic distillation. During the distillation the volume was kept constant. By the end of distillation the mixture was cooled to 20 °C. In case that no crystals were formed seeding crystals, obtained from a previous reaction, can be added as option. In order to remove crusts from the reactor wall, the mixture was heated to reflux and then cooled to 20 °C. The product, D10 L -malic acid, was collected by filtration and was dried under vacuum at 50 °C (42 % Yield. 1H-NMR, 400 MHz, d6-DMSO: see Figure 6).

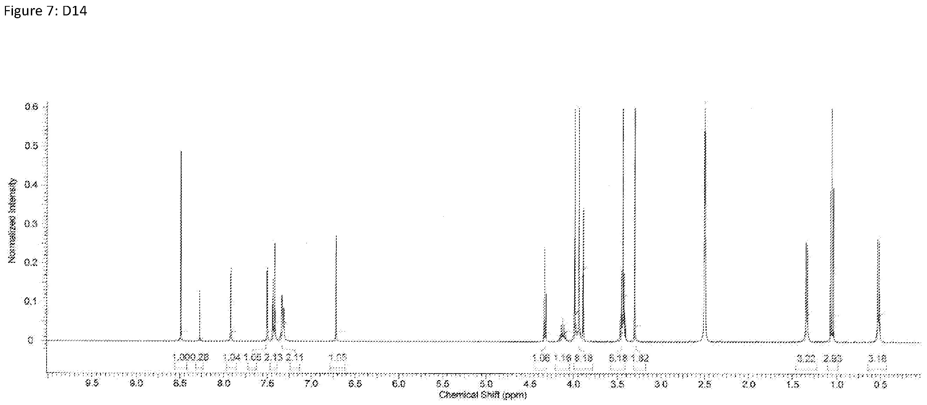

The reactor was charged with the mother liquor and solvent was removed by distillation. The organic layer was extracted with water. The solvent was switched to ethanol and, if necessary, seeding crystals of the compound D14, obtained from a previous reaction, were added. The mixture was cooled to 5 °C. Compound D14 was collected by filtration and dried under vacuum at 50 °C, to obtain compound D14 (47 % Yield, 1H-NMR, 400 MHz, d6-DMSO: see Figure 7).

Then a solution of compound D14 (40.00 g, 1.00 eq.) in 800 mL methanol was stirred at 65 °C for 15 min followed by addition of a 2.5 % aqueous solution of NaHCO₃ (44.5 g, 0.2 eq.). The reaction mixture was stirred for 24 hours at 65 °C and the racemization or formation of compound D9 was monitored by HPLC. The reaction mixture was then cooled to 22 °C and neutralized with sulfuric acid (3.38 g, 0.1095 eq.). The mixture was filtered over a Cap-filter and the solvent was removed. Ethanol was added and the mixture was cooled to 0 °C and stirred for 2 hours. The product was collected by filtration, was washed twice with ethanol and dried under vacuum at 50 °C to finally be used to produce compound D10 L-malic acid according to the procedure described above.

### Example 4: Hydrate formation (D10->D12)

A reactor was charged with 1.5 g of compound D10 L-malic acid, and the solid was dissolved in acetone (8.8 mL). The resulting solution was filtered at 25 °C and the filter was washed with acetone (2.5 mL). Then water (12.5 mL) was added at 25 °C to the solution within 1 hour. 2 mg of seed-crystals of the hydrate D12 were added to the resulting mixture. Then more water (26.3 mL) was added within 1 hour to the reaction mixture and the reaction was stirred at 25 °C for 4 hours. The resulting suspension was isolated through filtration and the filter cake was washed with 2 x 7.5mL of water at 25 °C. Finally, the product was dried under vacuum at 35 °C and 35 mbar for about 15 hours to give hydrate compound D12 (1.4 g).

### Example 5: Ethanol solvate formation (D10->D11)

A reactor was charged with compound D10 L-malic acid, (15.0 g, 1.00 eq.), ethyl acetate (300 mL) and water (75 mL). The suspension was heated to 50 °C and stirred until a clear biphasic solution was obtained. Then the reaction mixture was cooled to 25 °C to separate the organic/aqueous layers and the aqueous layer was removed. Then water (75 mL) was added and the mixture was stirred, and those steps were repeated twice. Finally the organic layer was filtered in order to obtain a clear solution. Solvent was removed by distillation and was replaced by ethanol. The solvent was removed to the final volume (75 mL), the mixture was cooled to 5 °C and stirred further. The product was collected by filtration and dried under vacuum at 50 °C to obtain the compound D11 as ethanolate (ethanol solvent) in a yield of 90%. 1H-NMR (400 MHz, DMSO-d6) delta ppm: 0.53 (d, *J*=6.78 Hz, 3H) 1.06 (t, *J*=6.90 Hz, 3H) 1.34 (d, *J*=6.78 Hz, 3H) 3.37- 3.53 (m, 5H) 3.95 (s, 3H) 3.99 (s, 3H) 4.05-4.18 (m, 1H) 4.34 (t, *J*=5.02 Hz, 1H) 6.73 (s, 1H) 7.33 (br d, *J*=8.28 Hz, 2H) 7.43 (d, *J*=7.90 Hz, 2H) 7.51 (d, *J*=2.76 Hz, 1H) 7.93 (d, *J*=2.76. 1H) 8.50 (s, 1H).

## Claims

1. A process for preparing a compound of formula (D7), or a salt thereof, or a solvate thereof, 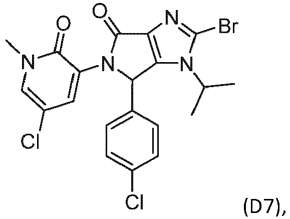 comprising the step of reacting a compound of formula (D6), or a salt thereof, or a solvate thereof, 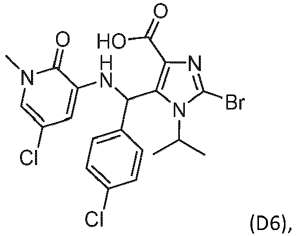 in a solvent in the presence of base and a coupling agent, wherein the coupling agent is N,N'-carbonyldiimidazole.

2. The process according to claim 1, wherein the solvent is one or more polar solvents selected from tetrahydrofuran, dimethylformamide, dimethylacetamide, *N*-methyl-2-pyrrolidone, dichloromethane, acetonitrile, ethyl acetate and ethanol.

3. The process according to any one of the preceding claims, wherein the base is one or more bases selected from tBuOK, LiHMDS, NaHMDS, MeONa, EtONa, tBuONa, K₂CO₃, Cs₂CO₃, K₃PO₄, DMAP, KOMe and KOEt, preferably the base is tBuOK.

4. The process according to claim 1, wherein the solvent is THF, the base is tBuOK, the coupling agent is *N,N'*-carbonyldiimidazole (CDI).

5. A process for preparing a compound of formula (I), 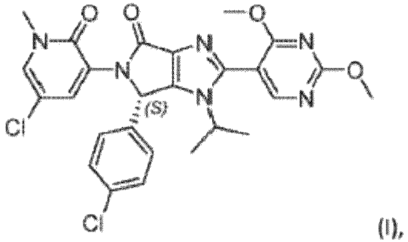 or a solvate including hydrate thereof, or a co-crystal, the process comprising the steps of:
i. preparing a compound of formula (D7), or a salt thereof, or a solvate thereof, according to any one of claims 1 to 4,
ii. preparing a compound of formula (D9), or a salt thereof, or a solvate thereof, 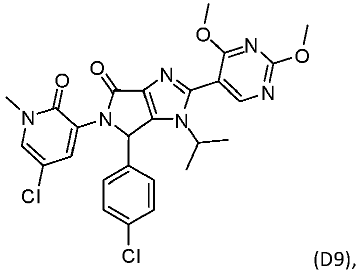 in a process comprising the step of reacting a compound of formula (D7), or a salt thereof, or a solvate thereof, 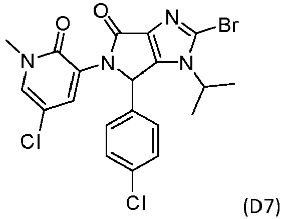 with a boronic acid of formula (D8), 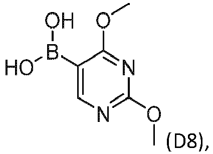 in a solvent in the presence of a metal-catalyst, a base and optionally a ligand, wherein the solution of (D8) is added to a solution of (D7) over a period of 2 to 8 hours,
iii. preparing a compound of formula (D10), 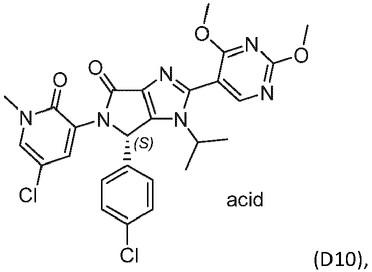 in a process comprising the step of
(a) reacting a compound of formula (D9), or a salt thereof, or a solvate thereof, in a solvent with an acid to obtain a mixture of compound of formula (D14), or a salt thereof, or a solvate thereof, and compound of formula (D10) 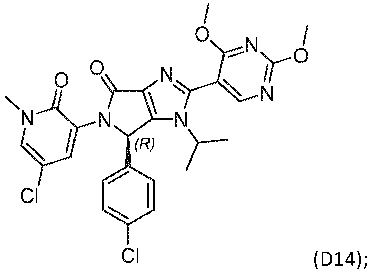 and optionally comprising the further steps of
(b) reacting (D14), or a salt thereof, or a solvate thereof, to obtain a compound of formula (D9), or a salt thereof, or a solvate thereof, in a solvent in the presence of a base; and
(c) reacting compound of formula (D9), or a salt thereof, or a solvate thereof, in a solvent in the presence of an acid to obtain compound (D10);
and optionally repeating the steps of (b) and (c) at least one time, and
iv. reacting the compound of formula (D10) to obtain a compound of formula (I), or a solvate including hydrate thereof, or a co-crystal thereof.

## Patentansprüche

1. Verfahren zur Herstellung einer Verbindung der Formel (D7) oder eines Salzes davon oder eines Solvats davon, 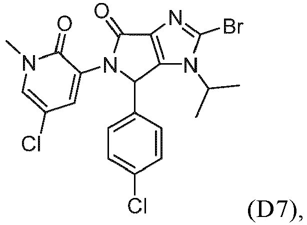 umfassend den Schritt des Umsetzens einer Verbindung der Formel (D6) oder eines Salzes davon oder eines Solvats davon, 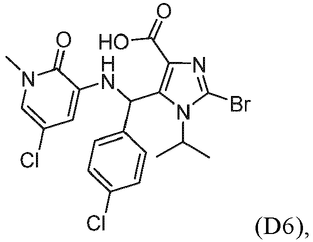 in einem Lösungsmittel in Gegenwart einer Base und eines Kupplungsmittels, wobei es sich bei dem Kupplungsmittel um *N,N'-*Carbonyldiimidazol handelt.

2. Verfahren nach Anspruch 1, wobei es sich bei dem Lösungsmittel um ein oder mehrere aus Tetrahydrofuran, Dimethylformamid, Dimethylacetamid, N-Methyl-2-pyrrolidon, Dichlormethan, Acetonitril, Essigsäureethylester und Ethanol ausgewählte polare Lösungsmittel handelt.

3. Verfahren nach einem der vorhergehenden Ansprüche, wobei es sich bei der Base um eine oder mehrere aus *t*BuOK, LiHMDS, NaHMDS, MeONa, EtONa, *t*BuONa, K₂CO₃, Cs₂CO₃, K₃PO₄, DMAP, KOMe und KOEt ausgewählte Basen handelt und es sich vorzugsweise bei der Base um *t*BuOK handelt.

4. Verfahren nach Anspruch 1, wobei es sich bei dem Lösungsmittel um THF handelt, es sich bei der Base um *t*BuOK handelt und es sich bei dem Kupplungsmittel um *N,N*'-Carbonyldiimidazol (CDI) handelt.

5. Verfahren zur Herstellung einer Verbindung der Formel (I), 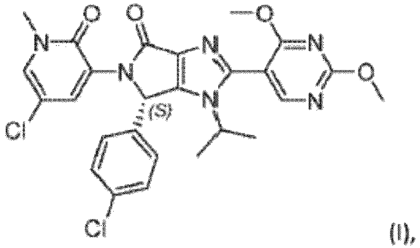 oder eines Solvats einschließlich Hydrats davon oder eines Cokristalls, wobei das Verfahren folgende Schritte umfasst:
i. Herstellen einer Verbindung der Formel (D7) oder eines Salzes davon oder eines Solvats davon nach einem der Ansprüche 1 bis 4,
ii. Herstellen einer Verbindung der Formel (D9) oder eines Salzes davon oder eines Solvats davon, 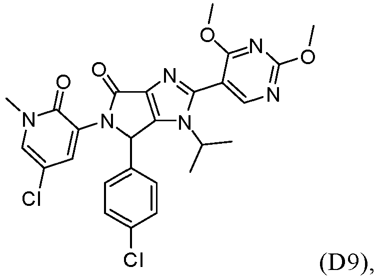 in einem Verfahren, das den Schritt des Umsetzens einer Verbindung der Formel (D7) oder eines Salzes davon oder eines Solvats davon, 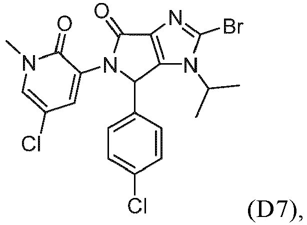 mit einer Boronsäure der Formel (D8), 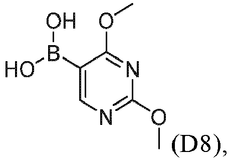 in einem Lösungsmittel in Gegenwart eines Metallkatalysators, einer Base und gegebenenfalls eines Liganden umfasst, wobei die Lösung von (D8) über einen Zeitraum von 2 bis 8 Stunden zu einer Lösung von (D7) gegeben wird,
iii. Herstellen einer Verbindung der Formel (D10), 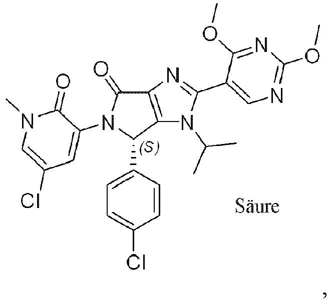 in einem Verfahren, das den folgenden Schritt umfasst:
(a) Umsetzen einer Verbindung der Formel (D9) oder eines Salzes davon oder eines Solvats davon in einem Lösungsmittel mit einer Säure zum Erhalt eines Gemischs der Verbindung der Formel (D14) oder eines Salzes davon oder eines Solvats davon und einer Verbindung der Formel (D10) 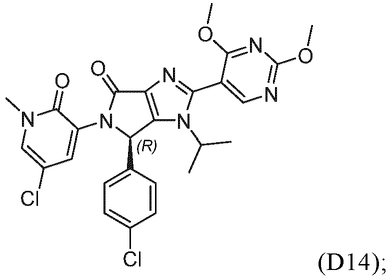 und gegebenenfalls die folgenden weiteren Schritte umfasst:
(b) Umsetzen von (D14) oder eines Salzes davon oder eines Solvats davon zum Erhalt einer Verbindung der Formel (D9) oder eines Salzes davon oder eines Solvats davon in einem Lösungsmittel in Gegenwart einer Base; und
(c) Umsetzen der Verbindung der Formel (D9) oder eines Salzes davon oder eines Solvats davon in einem Lösungsmittel in Gegenwart einer Säure zum Erhalt von Verbindung (D10);
und gegebenenfalls mindestens einmaliges Wiederholen der Schritte (b) und (c), und
iv. Umsetzen der Verbindung der Formel (D10) zum Erhalt einer Verbindung der Formel (I) oder eines Solvats einschließlich Hydrats davon oder eines Cokristalls davon.

## Revendications

1. Procédé pour la préparation d'un composé de formule (D7), ou d'un sel correspondant, ou d'un solvate correspondant, 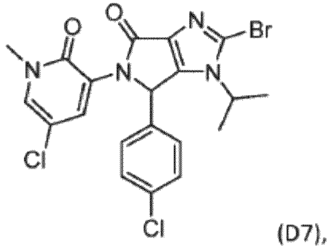 comprenant l'étape de mise en réaction d'un composé de formule (D6), ou d'un sel correspondant, ou d'un solvate correspondant, 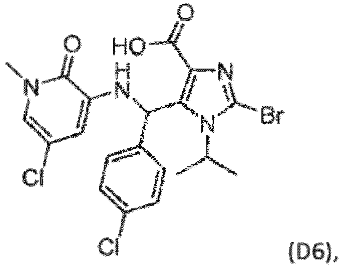 dans un solvant en la présence d'une base et un agent de couplage, l'agent de couplage étant le *N,N*'-carbonyldiimidazole.

2. Procédé selon la revendication 1, le solvant étant l'un ou plusieurs solvants polaires choisis parmi le tétrahydrofuranne, le diméthylformamide, le diméthylacétamide, la *N*-méthyl-2-pyrrolidone, le dichlorométhane, l'acétonitrile, l'acétate d'éthyle et l'éthanol.

3. Procédé selon l'une quelconque des revendications précédentes, la base étant l'une ou plusieurs bases choisies parmi *t*BuOK, LiHMDS, NaHMDS, MeONa, EtONa, tBuONa, K₂CO₃, Cs₂CO₃, K₃PO₄, DMAP, KOMe et KOEt, préférablement la base étant tBuOK.

4. Procédé selon la revendication 1, le solvant étant le THF, la base étant *t*BuOK, l'agent de couplage étant le *N,N*'-carbonyldiimidazole (CDI).

5. Procédé pour la préparation d'un composé de formule (I), 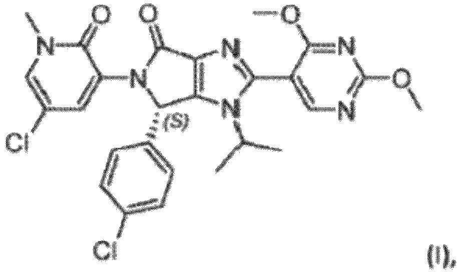 ou d'un solvate y compris un hydrate correspondant, ou d'un co-cristal, le procédé comprenant les étapes de :
i. préparation d'un composé de formule (D7), ou d'un sel correspondant, ou d'un solvate correspondant, selon l'une quelconque des revendications 1 à 4,
ii. préparation d'un composé de formule (D9), ou d'un sel correspondant, ou d'un solvate correspondant, 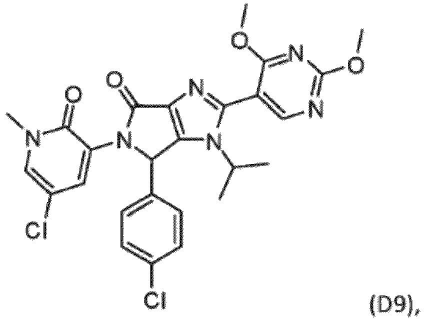 dans un procédé comprenant l'étape de mise en réaction d'un composé de formule (D7), ou d'un sel correspondant, ou d'un solvate correspondant, 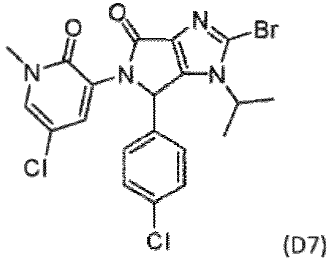 avec un acide boronique de formule (D8), 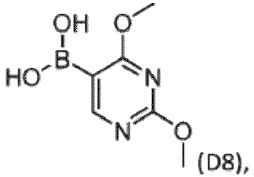 dans un solvant en la présence d'un catalyseur métallique, d'une base et éventuellement d'un ligand, la solution de (D8) étant ajoutée à une solution de (D7) sur une période de 2 à 8 heures,
iii. préparation d'un composé de formule (D10), 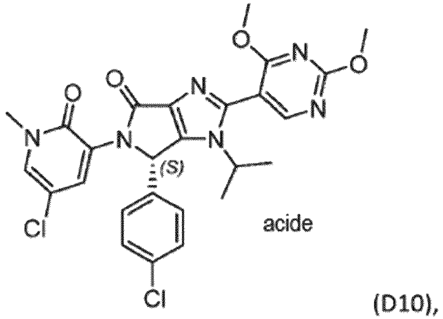 dans un procédé comprenant l'étape de
(a) mise en réaction d'un composé de formule (D9), ou d'un sel correspondant, ou d'un solvate correspondant, dans un solvant avec un acide pour obtenir un mélange d'un composé de formule (D14), ou d'un sel correspondant, ou d'un solvate correspondant, et d'un composé de formule (D10) 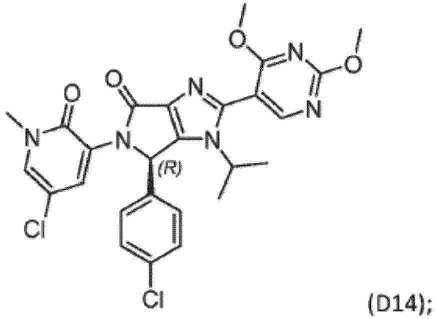 et comprenant éventuellement les étapes supplémentaires de
(b) mise en réaction de (D14), ou d'un sel correspondant, ou d'un solvate correspondant, pour obtenir un composé de formule (D9), ou un sel correspondant, ou un solvate correspondant, dans un solvant en la présence d'une base ; et
(c) mise en réaction du composé de formule (D9), ou d'un sel correspondant, ou d'un solvate correspondant, dans un solvant en la présence d'un acide pour obtenir le composé (D10) ;
et éventuellement la répétition des étapes de (b) et (c) au moins une fois, et
iv. mise en réaction du composé de formule (D10) pour obtenir un composé de formule (I), ou un solvate y compris un hydrate correspondant, ou un co-cristal correspondant.
